**Europäisches Patentamt**

**European Patent Office**

**Office européen des brevets**

(19)

(11) Veröffentlichungsnummer: **0 090 982**
**A1**

(12) # EUROPÄISCHE PATENTANMELDUNG

(21) Anmeldenummer: **83102681.0**

(22) Anmeldetag: **18.03.83**

(51) Int. Cl.³: **G 02 B 21/22**

(30) Priorität: **05.04.82 DE 3212691**

(43) Veröffentlichungstag der Anmeldung:
**12.10.83 Patentblatt 83/41**

(84) Benannte Vertragsstaaten:
**CH DE FR GB LI**

(71) Anmelder: **Firma Carl Zeiss**

**D-7920 Heidenheim (Brenz)(DE)**

(72) Erfinder: **Vogel, Albrecht**
**Hainbuchenweg 14**
**D-7082 Oberkochen(DE)**

(72) Erfinder: **Schulz, Kurt, Ing. grad.**
**Mozartweg 20**
**D-7082 Oberkochen(DE)**

(72) Erfinder: **Müller, Ortwin, Dipl.-phys.**
**Kolpingstrasse 34**
**D-7080 Aalen 1(DE)**

(54) **Prismenkompensator für stereoskopische Beobachtungsgeräte.**

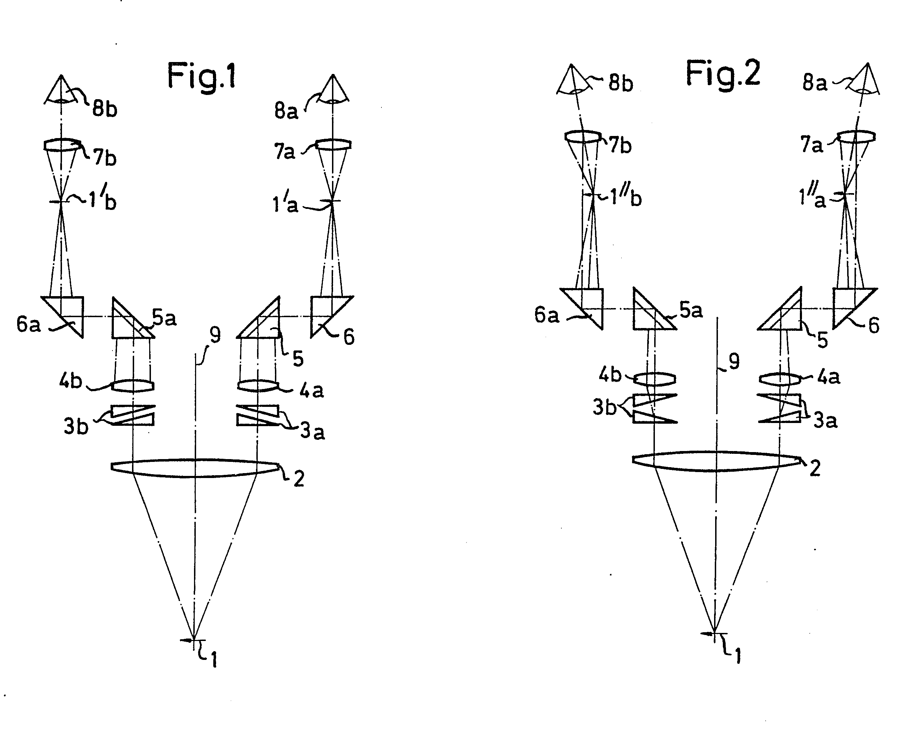

(57) Für stereoskopische Beobachtungsgeräte wird eine Kompensationsvorrichtung angegeben, die ein komfortables Binokularsehen für heterophore Benutzer ermöglicht. Die Vorrichtung besteht aus zwei Drehkeilsystemen (3a,3b), die synchron und kontinuierlich verstellbar und im parallelen Strahlengang hinter dem Hauptobjektiv (2) des stereoskopischen Beobachtungsgerätes angeordnet sind. Die Wirkungsweise der Kompensationsvorrichtung wird in Figur 2 demonstriert.

Fig.2

EP 0 090 982 A1

Prismenkompensator für stereoskopische Beobachtungsgeräte

Die Erfindung betrifft eine Kompensationsvorrichtung zur Verschmelzung zweier Teilbilder zu einem Seheindruck bei optischen Geräten für stereoskopische Beobachtung.

Stereoskopische Beobachtung ist insbesondere erwünscht und erforderlich bei ophthalmologischen und mikrochirurgischen Arbeiten, die mittels Spaltlampe oder Operationsmikroskop durchgeführt werden, die nach dem Prinzip der Fernrohrlupe arbeiten. Bei derartigen Geräten sind die Rohre des Binokulartubus parallel angeordnet, so daß bei einem Beobachter, der mit völlig entspannter Akkomodation in das Gerät blickt, die Fixierlinien des Augenpaares gleichfalls parallel ausgerichtet sind. Die vom optischen Gerät entworfenen beiden Teilbilder befinden sich beim Beobachter auf korrespondierenden Netzhautarealen und werden zu einem binokularen Seheindruck verschmolzen. Dieses "binokulare Einfachsehen" ist jedoch nicht bei allen Benutzern gewährleistet. Bei heterophoren Benutzern befinden sich die beiden Teilbilder auf sensorisch nicht korrespondierenden Netzhautarealen. In diesem Fall ist ein "binokulares Einfachsehen" als Voraussetzung für ein komfortables Binokularsehen, das bei ophthalmologischen Untersuchungen und mikrochirurgischen Eingriffen unabdingbar ist, nicht möglich oder zumindest erschwert.

Der Erfindung liegt deshalb die Aufgabe zugrunde, die in einem Operationsmikroskop oder in einer Spaltlampe erzeugten Einzelbilder für heterophore Beobachter und/oder bei akkomodativen Einflüssen des Beobachters auf sensorisch korrespondierende Netzhautareale zu lagern, um damit auch diesen Benutzern ein komfortables Binokularsehen zu gewährleisten.

Diese Aufgabe wird erfindungsgemäß dadurch gelöst, daß im parallelen Strahlengang hinter dem Hauptobjektiv oder einem zusätzlichen Galilei-Wechsler jedoch vor dem Binokulartubus des Gerätes für jeden Okularstrahlengang ein aus zwei Einzelprismen bestehendes Drehkeilsystem angeordnet ist. Zweckmäßigerweise sind beide Drehkeilsysteme synchron und kontinuierlich verstellbar.

In einem vorteilhaften Ausführungsbeispiel der Erfindung sind beide Drehkeilsysteme in einer Fassung montiert, die mittels Schwalbenverbindung in das Beobachtungsgerät einsetzbar ist.

Vorteilhafterweise werden Einzelprismen verwendet, welche bei einer Verdrehung von ca. 60° und einem Keilwinkel von ca. 80' eine prismatische Komponente von maximal zwei Prismendioptrien erzeugen.

Die mit der Erfindung erzielten Vorteile bestehen insbesondere darin, daß bei stereoskopischen Beobachtungsgeräten mit parallelen Tubusrohren auch heterophoren Beobachtern sowie Beobachtern, die infolge von akkomodativen Einflüssen Schwierigkeiten hinsichtlich der Unterdrückung von Doppelbildern haben, ein komfortables Binokularsehen ermöglicht wird.

Ein Ausführungsbeispiel der Erfindung ist in der Zeichnung dargestellt und wird im folgenden näher beschrieben. Es zeigen

Fig. 1    eine schematische Darstellung des optischen Strahlenganges in einem Operationsmikroskop mit einer Stellung der Drehkeilsysteme, in der keine Ablenkung der Einzelbilder stattfindet;

Fig. 2    den in Figur 1 dargestellten Strahlengang mit maximaler Ablenkung der Einzelbilder durch die Drehkeilsysteme;

Fig. 3    ein Ausführungsbeispiel einer Fassung für beide Drehkeilsysteme;

Fig. 4    eine erfindungsgemäße Kompensationsvorrichtung mit einer Spaltlampe in Teilmontage.

In der Fig. 1 ist das betrachtete Objekt mit der Kennziffer 1 bezeichnet. Mit 2 ist das Hauptobjektiv eines Operationsmikroskopes bezeichnet, mit 3a und 3b die im parallelen Strahlengang angeordneten Drehkeilpaare. 4a und 4b sind die Objektivlinsen der Okulartuben. Über die Umlenkprismen 5 und 6 bzw. 5a und 6a wird das Objekt 1 in 1a' und 1b' abgebildet und durch die Okularlinsen 7a, 7b von den Augen 8a, 8b betrachtet. In der in Fig. 1 gezeigten Stellung der Drehkeilpaare 3a, 3b, in der das dicke Ende des einen Keiles auf dem dünnen des anderen liegt, findet keine Ablenkung der Abbildungsstrahlen statt, da die Drehkeilsysteme in dieser Stellung einer planparallelen Platte entsprechen. Beim Beobachter sind die Fixierlinien der Augen 8a, 8b parallel ausgerichtet und die beiden Teilbilder befinden sich bei diesem Beobachter in dieser Konstellation der Drehkeilsysteme auf sensorisch korrespondierenden Netzhautarealen. In der in Fig. 2 dargestellten Konstellation der Drehkeilpaare liegen beide Keile jeweils mit ihren dicken Enden übereinander, die Abbildungsstrahlen erhalten dadurch die größtmögliche Ablenkung. Die Objektbilder 1a" und 1b" sind in diesem Fall zur optischen Hauptachse 9 des Gerätes hin verschoben. Beim Beobachter, bei dem der Einblick in den Binokulartubus einen Akkomodationsimpuls mit einer gleichzeitigen Konvergierung des Augenpaares ausgelöst hat, fallen bei der gezeigten Konstellation der Drehkeilsysteme die beiden verschobenen Einzelbilder auf korrespondierende Netzhautareale. Dadurch ist auch in diesem Fall ein komfortables Binokularsehen möglich.

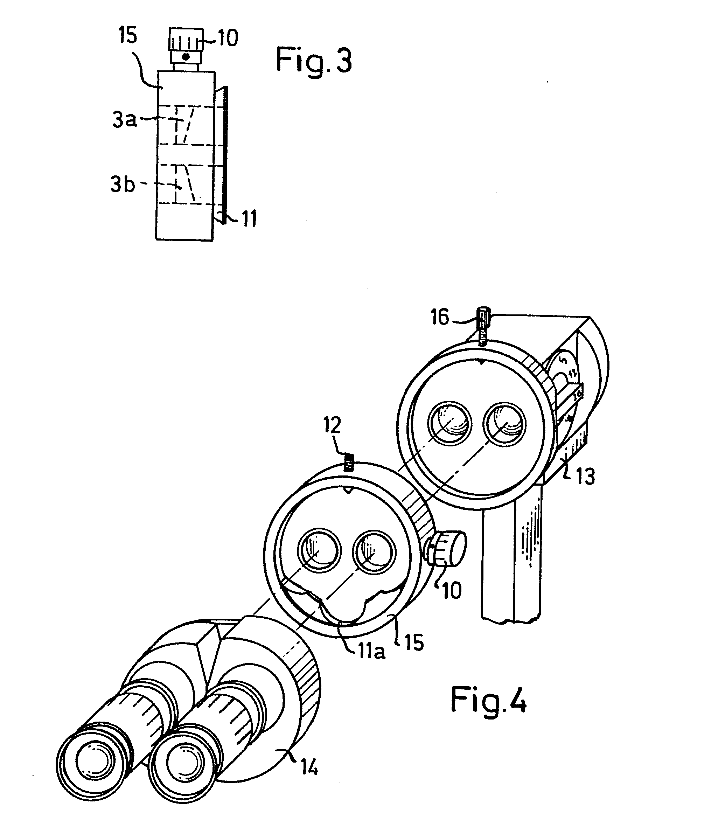

In der in Fig. 3 dargestellten Fassung 15 für die Drehkeilpaare 3a, 3b ist ein Drehknopf 10 sichtbar, mit dem die Verdrehung der Drehkeilpaare eingeleitet wird. Mit 11 ist eine Schwalbenschwanzaufnahme bezeichnet, die in ein entsprechendes Gegenstück im Grundkörper des Beobachtungsgerätes eingepaßt werden kann.

In der in Fig. 4 dargestellten Teilmontage der Keilfassung ist der Mikroskopkörper einer Spaltlampe mit 13 und der binokulare Beobachtungstubus der Spaltlampe mit 14 bezeichnet. Mit 12 ist eine Feststellschraube zur Befestigung der Fassung 15 am binokularen Beobachtungstubus 14 bezeichnet, der mit einem in der Abbildung der Fig. 4 nicht sichtbaren Vorsprung in die Vertiefung 11a eingreift. Eine entsprechende Feststellschraube 16 ist am Spaltlampenkörper 13 zur Befestigung der Fassung 15 angebracht.

0090982

Patentansprüche

1. Kompensationsvorrichtung zur Verschmelzung zweier Teilbilder zu einem Seheindruck bei optischen Geräten für stereoskopische Beobachtung, insbesondere Operationsmikroskopen und Spaltlampen, dadurch gekennzeichnet, daß im parallelen Strahlengang hinter dem Hauptobjektiv oder einem zusätzlichen Galilei-Wechsler jedoch vor dem Binokulartubus des Gerätes für jeden Okularstrahlengang ein aus zwei Einzelprismen bestehendes Drehkeilsystem angeordnet ist.

2. Kompensationsvorrichtung nach Anspruch 1, dadurch gekennzeichnet, daß beide Drehkeilsysteme synchron und kontinuierlich verstellbar sind.

3. Kompensationsvorrichtung nach Anspruch 2, dadurch gekennzeichnet, daß die Einzelprismen des Drehkeilsystems bei einer Verdrehung von 60° und einem Keilwinkel der Einzelprismen von 80' eine prismatische Komponente von maximal zwei Prismendioptrien erzeugen.

4. Kompensationsvorrichtung nach Anspruch 3, dadurch gekennzeichnet, daß beide Drehkeilsysteme in einer Fassung montiert sind, die mittels Schwalbenverbindung in das Beobachtungsgerät einsetzbar ist.

Fig.1

Fig.2

Fig.3

15
10
3a
3b
11

16

12

10

13

15

11a

14

Fig.4

Europäisches
Patentamt

EUROPÄISCHER RECHERCHENBERICHT

0090982
Nummer der Anmeldung

EP 83 10 2681

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y | US-A-4 188 097 (J.T. HOLLADAY) <br> * Spalte 2, Zeilen 53-55 * | 1,2 | G 02 B 21/22 |
| P,Y | DE-A-3 105 018 (C. ZEISS) <br> * Ansprüche 1, 3 * | 1,2 | |
| Y | CH-A- 415 099 (OPTISCHE WERKE RODENSTOCK) <br> * Anspruch 2; Figur * | 1,2 | |
| A | US-A-3 434 777 (L.J. SANTIROCCO) <br> * Zusammenfassung * | 4 | |

RECHERCHIERTE
SACHGEBIETE (Int. Cl. ³)

A 61 B 3/10
G 02 B 21/20
G 02 B 21/22

Der vorliegende Recherchenbericht wurde für alle Patentansprüche erstellt.

| Recherchenort <br> BERLIN | Abschlußdatum der Recherche <br> 06-06-1983 | Prüfer <br> FUCHS R |
|---|---|---|

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82